(19) Europäisches Patentamt
European Patent Office
Office européen des brevets

(11) Publication number: **0 318 138 B1**

# (12) EUROPEAN PATENT SPECIFICATION

(45) Date of publication of patent specification: **24.11.93** (51) Int. Cl.$^5$: **A61K 7/16**

(21) Application number: **88308990.6**

(22) Date of filing: **28.09.88**

## (54) Use of inorganic fluorides and nitrogen heterocyclics for enhancement of fluoride uptake in teeth.

(30) Priority: **25.11.87 JP 296374/87**

(43) Date of publication of application:
**31.05.89 Bulletin 89/22**

(45) Publication of the grant of the patent:
**24.11.93 Bulletin 93/47**

(84) Designated Contracting States:
**DE FR GB IT NL**

(56) References cited:
**EP-A- 0 049 830**
**US-A- 4 515 771**

(73) Proprietor: **SHISEIDO COMPANY LIMITED**
**5-5 Ginza 7-chome**
**Chuo-ku Tokyo(JP)**

(72) Inventor: **Tanaka, Kumiko c/oShiseido Laboratories**
**1050, Nippa-cho**
**Kohoku-ku**
**Yokohama-shi Kanagawa(JP)**
Inventor: **Fujii, Seishiro c/oShiseido Laboratories**
**1050, Nippa-cho**
**Kohoku-ku**
**Yokohama-shi Kanagawa(JP)**

(74) Representative: **Newby, John Ross et al**
**J.Y. & G.W. Johnson**
**Furnival House**
**14/18 High Holborn**
**London WC1V 6DE (GB)**

EP 0 318 138 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the use of inorganic fluoride compounds and nitrogen heterocyclic compounds for the manufacture of a dentifrice or mouthwash composition for enhancement of the uptake of fluoride in the tooth in comparison with a conventional dentifrice or mouthwash composition.

Fluoride compounds are widely known to have an effect in preventing caries in teeth. Caries is a phenomenon whereby a tooth is decalcified by the action of organic acids such as lactic acid, formed from decomposition of sugars such as sucrose, by microorganisms present in the oral cavity, mainly Streptococcus mutans.

Hydroxyapatite is a main component of tooth enamel, and the uptake of fluoride ions into the crystal lattice of hydroxyapatite is easily performed and the fluoride ion enhances the crystallinity of the lattice. As a result, the tooth is reinforced, the enamel becomes more resistant to decalcification by organic acids formed by the microorganisms present in the oval cavity, and thus caries may be prevented. It is also believed that the uptake of the fluoride ion is more easily effected into a lesion of incipient caries (wherein some decalcification proceeds) than into sound enamel, and thus enhances recalcification and inhibits the progress of caries.

To prevent caries, a fluoride compound such as sodium fluoride, sodium monofluorophosphate, or stannous fluoride has hitherto been added to tap water, or to a dentifrice, and topically applied to the tooth surface. Further, mouth washes have been formulated with the fluoride compound.

Nevertheless, since caries is still widespread, conventional methods wherein only the fluoride compound is employed do not satisfactorily prevent caries, and there is a demand for a more effective method of caries prevention.

US-A-4,515,771 discloses an oral composition for the preventive treatment of dental diseases, the composition including pyridoxine hydrochloride, an anti-inflammatory agent, a gelling agent and optionally sodium fluoride. The oral composition is intended to be delivered and retained below the gum line where it may effectively come into contact with the types of bacteria which typically become trapped and cause distress beneath the gum line so as to prevent initiation and progress of bacterially induced dental diseases such as periodontal disease. Special equipment is described for applying the composition to the correct spot.

EP-A-0,049,830 discloses an oral composition comprising hexetidine or its derivatives and zinc salt such as zinc fluoride. The hexetidine is said to be an anti-bacterial agent and exhibits synergy with zinc.

After conducting intensive research into the more effective prevention of caries, the present inventors found that, when a conventional inorganic fluoride compound is used in combination with one or more particular substituted nitrogen-heterocyclic compounds, the uptake of the fluoride ion into the tooth, specifically into hydroxyapatite, can be more easily effected, and thus the reinforcement action of the fluoride ion to the tooth is enhanced. The present invention is based on the above discovery.

The present invention therefore provides the use of (i) at least one fluoride compound and (ii) at least one nitrogen heterocyclic compound selected from pyridoxine, pyridoxal, pyridoxamine, and salts thereof; 2- or 3-pyridyl carbinol; thiamine and salts thereof; histidine and salts thereof; and nicotinic acid and nicotinic acid amide; for the manufacture of a dentifrice or mouth wash composition for enhancement of the uptake of fluoride in the tooth.

The inorganic fluoride compounds which may be used in the present invention are compounds containing a fluoride which is prevailingly present in the biological field. The fluoride compounds include alkali metal fluorides such as sodium fluoride, potassium fluoride, lithium fluoride, or cesium fluoride; zirconium fluoride, ammonium fluoride, stannous fluoride; alkali metal monofluorophosphates such as sodium monofluorophosphate, or potassium monofluorophosphate; and alkali metal titanium fluorides such as sodium titanium fluoride or potassium titanium fluoride; the above fluoride compounds may be used singly or in combination. From the viewpoint of oral application, sodium fluoride, potassium fluoride, ammonium fluoride, stannous fluoride, sodium monofluorophosphate or potassium monofluorophosphate is preferable.

The fluoride compound is generally incorporated in the dentifrice or mouthwash composition in an amount of from 50 - 10,000 ppm ($\mu$g/g), preferably 200 - 10,000 ppm, as the total fluorine concentration in the composition. If the amount is less than 50 ppm, a desired reinforcement action cannot be obtained. Although the fluoride compound may be used in the amount of more than 10,000 ppm, the desired effect does not increase as the amount increases, and thus it is not economical. When producing the dentifrice, a total fluorine concentration of 1,000 ppm or less is preferable.

Most of the nitrogen-heterocyclic compounds used are pyridine derivatives of the formula

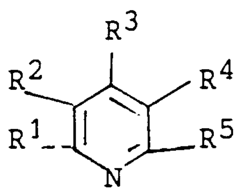

(1)

and are selected from pyridoxine ($R^1 = CH_3$ , $R^2 = OH$, $R^3 = CH_2OH$, $R^4 = CH_2OH$ and $R^5 = H$), pyridoxal ($R^1 = CH_3$ , $R^2 = OH$, $R^3 = CHO$, $R^4 = CH_2OH$ and $R^5 = H$), pyridoxamine ($R^1 = CH_3$ , $R^2 = OH$, $R^3 = CH_2NH_2$, $R^4 = CH_2OH$ and $R^5 = H$), and salts thereof (e.g. hydrochloride); 3-pyridylcarbinol ($R^1 = R^2 = R^3 = R^5 = H$, $R^4 = CH_2OH$) or 2-pyridylcarbinol ($R^1 = R^2 = R^3 = R^4 = H$, $R^5 = CH_2OH$); nicotinic acid ($R^1 = R^2 = R^3 = R^5 = H$, $R^4 = COOH$), and nicotinic acid amide ($R^1 = R^2 = R^3 = R^5 = H$, $R^4 = CONH_2$).

Of those monocyclic compounds, pyridoxine hydrochloride, 3-pyridylcarbinol and 2-pyridylcarbinol are preferable.

The other nitrogen-heterocyclic compounds used have the formula

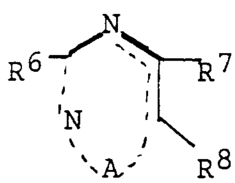

(2)

and are selected from histidine and salts thereof, preferably histidine hydrochloride, and thiamine and salts thereof, preferably thiamine hydrochloride.

The above heterocyclic compounds may be used singly or in combination.

In the dentifrices and mouthwash compositions the molar ratio of the nitrogen-heterocyclic compound to the fluoride compound is generally 1/50 to 10/1, preferably 1/10 to 5/1, more preferably 1/10 to 3/1. If the molar ratio is less than 1/50, the desired reinforcement action of the fluoride cannot be obtained, whereas if the ratio is more than 10/1, the effect does not increase as the amount increases, and thus is not economical.

In addition to the above two essential components, the dentifrice or mouth wash composition may contain various additives. The kinds of additives depend on the types of composition. Examples of the additives are polishing agents such as calcium phosphate (dibasic) dihydrate or anhydrate thereof, calcium phosphate (monobasic), calcium phosphate (tribasic), calcium carbonate, calcium pyrophosphate, titanium oxide, aluminum hydroxide, aluminum oxide, silica polishing agents (e.g. amorphous silica, crystalline silica, complex of alkali metal silicic anhydride), aluminum silicate, insoluble sodium metaphosphate, insoluble potassium metaphosphate, insoluble calcium polyphosphate, magnesium phosphate (tribasic), magnesium carbonate, magnesium sulfate, calcium sulfate, methyl polymethacrylate, bentonite, zirconium silicate, hydroxyapatite or synthetic polymer; wetting agents such as glycerol, sorbitol, propylene glycol, polyethylene glycol, ethylene glycol, 1,3-butylene glycol, xylitol, maltitol, or lactitol; thickening agents such as carboxymethyl cellulose, methyl cellulose, hydroxyethyl cellulose, sodium carboxymethylhydroxyethyl cellulose, sodium alginate, carrageenan, sodium polyacrylate, gum arabic, xanthan gum, tragacanth gum, karaya gum, polyvinyl alcohol, carboxyvinyl polymer, or polyvinyl pyrrolidone; anionic surface active agents such as water soluble salts of $C_{8-18}$ alkyl sulfate (e.g. sodium lauryl sulfate, sodium myristyl sulfate), soluble higher fatty acid monoglyceride sulfate wherein the fatty acid has 10-18 carbon atoms (e.g. sodium lauryl monoglyceride sulfate, sodium coconut oil fatty acid monoglyceride sulfate, sodium higher fatty acid monoglyceride sulfate), $\alpha$-olefin sulfate, paraffin sulfate, sodium salt of N-methyl-N-palmitoyl tauride, sodium N-lauryl sarcosinate, sodium salt of N-lauryl-$\beta$-alanine; nonionic surface active agents such as fatty acid alkanolamide (e.g. lauric acid diethylamide), sucrose fatty acid ester (e.g. sucrose mono- or dilaurate), polyoxyethylene sorbitan fatty acid ester, polyoxyethylene fatty acid ester, polyoxyethylene hardened castor oil derivative, lactose fatty acid ester, maltitol fatty acid ester, or polyoxyethylene polyoxypropylene block copolymer; cationic or amphoteric surface active agents; oils such as higher alcohol or wax; lower alcohols; edulcorants such as sodium saccharin, stevioside, neohesperidin, dihydrochalcone, glycyrrhizin, perillartine,

p-methoxycinnamic aldehyde; essential oils such as peppermint, spearmint, or fennel; perfumes such as l-menthol, carvone, eugenol, anethol or other perfume base; colorants; preservatives; antimold agents; antioxidants; and water. Further, the compositions may contain ingredients usually incorporated in dentifrice and mouth wash composition, for example dextranase, protease, lytic enzyme, mutase, mutastein, sorbic acid, alexin, β-glycyrrhetinic acid, hinokitiol, dihydrocholesterol, epidihyrocholesterol, alkyl glycine, alkyldiamino-ethylyglycine salt, allantoin, ϵ-aminocaproic acid, tranexamic acid, azulene, other vitamins, water soluble salts of phosphoric acid (mono- or dibasic), quaternary ammonium compounds (e.g. cetylpyridinium chloride), sodium chloride, or active components (e.g. crude drug extracts).

The dentifrice can be, e.g.,a paste dentifrice, a powdery dentifrice, a wet tooth powder, or a liquid tooth powder.

The pH value of the composition is not critical, but is preferably 3-10, more preferably 4-9, most preferably 5-7.

## EXPERIMENTS

The caries prevention effect of the compositions will be illustrated hereinafter.

### Experiment 1

Sodium fluoride and the heterocyclic compounds or the fatty acid ester (for comparison) listed in Tables 1 to 3 were added to a distilled water in the amounts shown in Tables 1 to 3, and the pH of the solution was adjusted to 4.5 with dilute hydrochloride or sodium hydroxide to form several preparations. Synthetic hydroxyapatite powder was incubated in each of the preparations at 37°C for 5 minutes and in a calcifying solution at 37°C for 60 minutes, and then filtrated and dried. The fluorine concentration in the treated synthetic hydroxyapatite powder was determined by microdiffusion analysis, and the amount of dissolved calcium after incubating the treated powder in an acetate buffer at 37°C for 60 minutes was measured. The inhibition (%) of decalcification was then calculated by the following equation, to evaluate the acid resistance thereof.

$$\text{Inhibition of decalcification (\%)} = \left(1 - \frac{\text{Amount of dissolved Ca of tested group}}{\text{Amount of dissolved Ca of control group}}\right) \times 100$$

The results are shown in Tables 1 to 3.

Table 1

| Sodium fluoride (%) | Pyridoxine hydrochloride (%) | Fluorine concentration (ppm) | Inhibition of decalcification (%) |
|---|---|---|---|
| 0 | 0 | 34 | - |
| (distilled water) | | | |
| 0.2 | O | 4910 | 40.9 |
| 0.2 | 0.2 | 14071 | 61.2 |
| (molar ratio about 1:0.2) | | | |
| 0.2 | 1.0 | 20320 | 72.1 |
| (molar ratio about 1:1) | | | |
| 0.2 | 2.0 | 23620 | 73.9 |
| (molar ratio about 1:2) | | | |
| 0.2 | 3.0 | 27120 | 74.4 |
| (molar ratio about 1:3) | | | |

Table 2

| | Fluorine concentration (ppm) | Inhibition of decalcification (%) |
|---|---|---|
| Distilled water | 34 | - |
| Sodium fluoride 0.2% | 4910 | 40.9 |
| Sodium fluoride 0.2% + 3-pyridylcarbinol 0.52% (molar ratio about 1:1) | 21187 | 72.1 |
| Sodium fluoride 0.2% + nicotinic acid 0.59% (molar ratio about 1:1) | 18765 | 70.1 |
| Sodium fluoride 0.2% + nicotinic acid amide 0.58% (molar ratio about 1:1) | 9891 | 53.0 |
| Sodium fluoride 0.2% + methyl nicotinate 0.65% (molar ratio about 1:1) | 16255 | 65.0 |

Table 3

| | Fluorine concentration (ppm) | Inhibition of decalcification (%) |
|---|---|---|
| Distilled water | 33 | - |
| Sodium fluoride 0.2% | 4796 | 40.9 |
| Sodium fluoride 0.2% + thiamine hydrochloride 1.59% (molar ratio about 1:1) | 22840 | 75.8 |
| Sodium fluoride 0.2% + acrinol 1.70% (molar ratio about 1:1) | 13026 | 68.2 |
| Sodium fluoride 0.2% + histidine hydrochloride 0.96% (molar ratio about 1:1) | 11668 | 62.3 |
| Sodium fluoride 0.2% + sucrose fatty acid ester 3.12% (molar ratio about 1:1) | 6921 | 48.0 |

The above data indicates that the synthetic hydroxyapatite includes more fluorine and has a higher resistance to acid when sodium fluoride is used in combination with the fatty acid ester, in particular the above heterocyclic compounds, in comparison with the use of sodium fluoride alone, and accordingly, the

reinforcement of the effect of sodium fluoride by the heterocyclic compounds was confirmed.

Experiment 2

A fluoride compound, and pyridoxine hydrochloride, 3-pyridylcarbinol or thiamine hydrochloride were added to distilled water, in amounts shown in table 4 to produce preparations. A bovine tooth (which had been ground to remove 200 µm of the surface and expose the enamel surface) was incubated in each preparation at 37°C for 5 minutes and in a calcifying solution at 37°C for 60 minutes, and then washed with tap water and dried. The fluorine concentration in the enamel of the fluoride-treated bovine tooth was determined by enamel biopsy. The fluoride-treated bovine tooth was dipped in an acidic gelatin gel prepared by the procedure disclosed in Leon M. Silverstone, Caries Research, 1:261-274, 1967, then after 4, 8, and 15 weeks, the amount of decalcification was observed by microradiography, and the acid resistance was evaluated by the following ratings.

◎ Decalcification substantially inhibited.
o Decalcification acceptably inhibited.
Δ Considerable decalcification.
x ... No inhibition of decalcification.

The results are shown in Table 4.

Table 4

| Preparation | | Distilled water | Sodium fluoride (2%) | Sodium fluoride (2%) + pyridoxine hydrochloride (10%) (molar ratio 1:1) | Sodium fluoride (2%) + 3-pyridylcarbinol (5.2%) (molar ratio 1:1) | Sodium fluoride (2%) + thiamine hydrochloride (15%) (molar ratio 1:1) |
|---|---|---|---|---|---|---|
| F conc. in enamel | 1st Etch | 38 ppm (10.2 μm) | 498 ppm (9.2 μm) | 14988 ppm (1.8 μm) | 15033 ppm (1.7 μm) | 16001 ppm (1.5 μm) |
| | 2nd | 35 ppm (21.5 μm) | 309 ppm (20.0 μm) | 7859 ppm (5.1 μm) | 8001 ppm (4.9 μm) | 7569 ppm (5.1 μm) |
| | 3rd | 34 ppm (33.7 μm) | 222 ppm (32.6 μm) | 4522 ppm (9.3 μm) | 4655 ppm (9.0 μm) | 4431 ppm (9.0 μm) |
| | 4th | 33 ppm (46.3 μm) | 178 ppm (46.5 μm) | 3225 ppm (14.7 μm) | 3211 ppm (14.1 μm) | 2988 ppm (14.1 μm) |
| | 5th | 30 ppm (60.0 μm) | 139.pp, (61.6 μm) | 2985 ppm (22.6 μm) | 3102 ppm (21.5 μm) | 2847 ppm (22.4 μm) |
| Inhibition of decalcification | 4W | x | o | ◎ | ◎ | ◎ |
| | 8W | x | Δ | ◎ | ◎ | ◎ |
| | 15W | x | x | o | o | o |

The above data indicates that the bovine enamel includes more fluorine and has a higher inhibition of decalcification when sodium fluoride is used in combination with the above heterocyclic compounds, in comparison with the use of sodium fluoride alone, and accordingly, the reinforcement of the effect of

sodium fluoride by the heterocyclic compounds was confirmed.

It is obvious from Experiments 1 and 2 that the present oral composition is a very useful composition for enhancing a conventional effect of preventing caries with fluoride, by employing the fluoride compound in combination with one or more nitrogen-heterocyclic compounds.

## EXAMPLES

The present invention now will be further illustrated by the following Examples. In the following Examples, the amounts of the ingredients incorporated are percentage by weight.

### Example 1: Paste dentifrice

In accordance with a conventional procedure, a paste dentifrice was prepared from the following composition:

| | |
|---|---|
| Calcium phosphate (dibasic) dihydrate | 40.0 |
| Glycerol | 10.0 |
| Sorbitol | 10.0 |
| Sodium carboxymethyl cellulose | 1.5 |
| Sodium lauryl sulfate | 1.5 |
| Sodium saccharin | 0.1 |
| Perfume | 1.0 |
| Sodium monofluorophosphate | 0.76 |
| Pyridoxine hydrochloride | 1.0 |
| Purified water | q.s. |

### Example 2

The procedure of Example 1 was repeated, except that 0.52% by weight of 3-pyridylcarbinol was used instead of 1.0% by weight of pyridoxin hydrochloride.

### Example 3

The procedure of Example 1 was repeated, except that 1.5% by weight of thiamine hydrochloride was used instead of 1.0% by weight of pyridoxin hydrochloride.

### Comparative Example 1

The procedure of Example 1 was repeated, except that pyridoxin hydrochloride was omitted.

### Comparative Example 2

The procedure of Example 1 was repeated, except that sodium monofluorophosphate and pyridoxin hydrochloride were omitted.

The paste dentifrices of Examples 1 to 3 and Comparative Examples 1 to 2 were diluted 10 times with distilled water, and a human tooth was incubated in each diluted liquid at 37 °C for 60 minutes, thoroughly washed, and dried. An enamel powder was obtained from the treated human tooth. The fluorine concentration in the enamel powder was determined by a microdiffusion analysis, and the amount of dissolved calcium after incubating the treated human tooth in an acetate buffer at 37 °C for 60 minutes was measured. The inhibition (%) of decalcification was calculated by the following equation, to evaluate the acid resistance thereof.

$$\text{Inhibition of decalcification (\%)} = \left(1 - \frac{\text{Amount of dissolved Ca of tested group}}{\text{Amount of dissolved Ca of control group}}\right) \times 100$$

The results are shown in Table 5.

Table 5

| Paste dentifrice | Fluorine concentration (ppm) | Inhibition of decalcification (%) |
|---|---|---|
| Example 1 | 1428 | 45.8 |
| Example 2 | 1563 | 47.5 |
| Example 3 | 1623 | 50.5 |
| Comparative Example 1 | 620 | 29.1 |
| Comparative Example 2 | 42 | 0.1 |

It is apparent from Table 5 that the paste dentifrices according to the present invention exhibit a very high fluorine trapping effect and inhibition of decalcification, in comparison with the comparative paste dentifrices.

Example 4: Paste dentifrice

In accordance with a conventional procedure, a paste dentifrice was prepared from the following composition:

| | |
|---|---|
| Silicic acid anhydride | 20.0 |
| Sorbitol | 50.0 |
| Carrageenan | 0.5 |
| Sodium carboxymethyl cellulose | 1.0 |
| Sodium lauryl sulfate | 1.8 |
| Sodium saccharin | 0.08 |
| Methyl paraoxybenzoate | 0.2 |
| Perfume | 0.9 |
| Sodium fluoride | 0.2 |
| Pyridoxine hydrochloride | 0.2 |
| Purified water | q.s. |

The above procedure was repeated, except that 0.2% by weight of pyridoxine hydrochloride was replaced by 0.5% by weight of nicotinic acid or 1.6% by weight of acrinol.

Example 5: Paste dentifrice

In accordance with a conventional procedure, a paste dentifrice was prepared from the following composition:

| | |
|---|---|
| Sorbitol | 20.0 |
| Silicic acid anhydride | 50.0 |
| Sodium polyacrylate | 0.7 |
| Sodium lauryl sulfate | 1.8 |
| Triethanolamine | 0.5 |
| Sodium saccharin | 0.1 |
| Sodium paraoxybenzoate | 0.2 |
| Perfume | 0.9 |
| Sodium monofluorophosphate | 0.76 |
| Pyridoxine hydrochloride | 0.2 |
| Allantoin | 1.0 |
| Purified water | q.s. |

The above procedure was repeated, except that 0.2% by weight of pyridoxine hydrochloride was replaced by 0.3% by weight of nicotinic acid amide, or 1.0% by weight of histidine hydrochloride and 0.2% by weight of pyridoxine hydrochloride.

Example 6: Mouth wash

In accordance with a conventional procedure, a mouth wash was prepared from the following composition:

| | |
|---|---|
| Ethylalcohol | 10.0 |
| Sodium saccharin | 0.05 |
| Perfume | 0.8 |
| Polyoxyethylene (20 mole) sorbitan lauric acid ester | 1.0 |
| Sodium fluoride | 0.02 |
| Pyridoxine hydrochloride | 0.02 |
| Purified water | q.s. |

The above procedure was repeated, except that 0.02% by weight of pyridoxine hydrochloride was replaced by 1.0% by weight of nicotinic acid amide, or 0.5% by weight of thiamine hydrochloride.

Example 7: Mouth detergent

In accordance with a conventional procedure, a mouth detergent was prepared from the following composition:

| | |
|---|---|
| Sodium fluoride | 0.05 |
| Pyridoxine hydrochloride | 0.02 |
| Sodium phosphate (dibasic) | 0.3 |
| Sodium phosphate (monobasic) | 0.7 |
| Perfume | 0.5 |
| Sodium saccharin | 0.02 |
| Purified water | q.s. |

The above procedure was repeated, except that 0.02% by weight of pyridoxine hydrochloride was replaced by 0.5% by weight of 3-pyridylcarbinol, or 0.2% by weight of thiamine hydrochloride.

Example 8: Topical application

In accordance with a conventional procedure, a topical application was prepared from the following composition:

| | |
|---|---|
| Sodium fluoride | 2.0 |
| Pyridoxine hydrochloride | 1.0 |
| Perfume | 0.5 |
| Sodium saccharin | 0.02 |
| Purified water | q.s. |

The above procedure was repeated, except that 1.0% by weight of pyridoxine hydrochloride was replaced by 1.0% by weight of 3-pyridylcarbinol, or 5.0% by weight of thiamine hydrochloride.

Example 9: Oral refrigerant (spray type)

In accordance with a conventional procedure, an oral refrigerant was prepared from the following composition:

| | |
|---|---|
| Ethylalcohol | 40.0 |
| Sodium saccharin | 0.1 |
| Sorbitol | 10.0 |
| Perfume | 1.0 |
| Polyoxyethylene (60 mole) hardened castor oil | 0.7 |
| Chlorohexdine hydrochloride | 0.05 |
| Sodium fluoride | 0.05 |
| Pyridoxine hydrochloride | 0.5 |
| Purified water | q.s. |

The above procedure was repeated, except that 0.5% by weight of pyridoxine hydrochloride was replaced by 0.5% by weight of methyl nicotinate, or 0.2% by weight of thiamine hydrochloride.

Example 10: Chewing gum

In accordance with a conventional procedure, a chewing gum was prepared from the following composition:

| | |
|---|---|
| Gum base | 25.0 |
| Calcium carbonate | 2.0 |
| Perfume | 1.0 |
| Copper chlorophyll | 0.05 |
| Sodium fluoride | 0.2 |
| Pyridoxine hydrochloride | 1.0 |
| Sorbitol | q.s. |

The above procedure was repeated, except that 1.0% by weight of pyridoxine hydrochloride was replaced by 0.1% by weight of ethyl nicotinate, or 1.0% by weight of thiamine hydrochloride.

**Claims**

**1.** The use of
(i) at least one inorganic fluoride compound and
(ii) at least one nitrogen-heterocyclic compound selected from pyridoxine, pyridoxal, pyridoxamine, and salts thereof; 2- or 3- pyridyl carbinol; thiamine and salts thereof; histidine and salts thereof; and nicotinic acid and nicotinic acid amide;
for the manufacture of a dentifrice or mouth wash composition for enhancement of the uptake of Fluoride in the tooth.

**2.** The use according to claim 1, wherein the nitrogen-heterocyclic compound is at least one compound selected from pyridoxine, pyridoxal, pyridoxamine and salts thereof.

3. The use according to claim 1, wherein the nitrogen-heterocyclic compound is 2- or 3- pyridyl carbinol.

4. The use according to claim 1, wherein the nitrogen-heterocyclic compound is at least one compound selected from thiamine and salts thereof.

5. The use according to claim 1, wherein the nitrogen-heterocyclic compound is at least one compound selected from histidine and salts thereof.

6. The use according to claim 1, wherein the nitrogen-heterocyclic compound is at least one compound selected from nicotinic acid and nicotinic acid amide.

7. The use according to any one of claims 1 to 6, wherein the amount of the fluoride compound incorporated is from 50 to 10,000 µg per gram of the composition as a total fluorine concentration, and the molar ratio of the nitrogen-heterocyclic compound to the fluoride compound is from 1/50 to 10/1.

8. The use according to any one of claims 1 to 7, said composition further comprising (iii) at least one additive selected from a polishing agent, wetting agent, thickening agent, surface-active agent, oil, edulcorant, essential oil, perfume colorant, preservative, antimold agent, antioxidant and water.

## Patentansprüche

1. Verwendung von
   (i) mindestens einer anorganischen Fluorid-Verbindung und
   (ii) mindestens einer Stickstoff enthaltenden heterocyclischen Verbindung, ausgewählt aus Pyridoxin, Pyridoxal, Pyridoxamin und Salzen hiervon; 2- oder 3-Pyridylcarbinol; Thiamin und Salzen hiervon; Histidin und Salzen hiervon; sowie Nikotinsäure und Nikotinsäureamid;
   zur Herstellung eines Zahnputzmittels oder eines Mundwaschmittels zur Erhöhung der Aufnahme von Fluorid im Zahn.

2. Verwendung nach Anspruch 1, bei der die Stickstoff enthaltende heterocyclische Verbindung mindestens eine Verbindung ausgewählt aus Pyridoxin, Pyridoxal, Pyridoxamin und Salzen hiervon ist.

3. Verwendung nach Anspruch 1, bei der die Stickstoff enthaltende heterocyclische Verbindung 2- oder 3-Pyridylcarbinol ist.

4. Verwendung nach Anspruch 1, bei der die Stickstoff enthaltende heterocyclische Verbindung mindestens eine Verbindung ausgewählt aus Thiamin und Salzen hiervon ist.

5. Verwendung nach Anspruch 1, bei der die Stickstoff enthaltende heterocyclische Verbindung mindestens eine Verbindung ausgewählt aus Histidin und Salzen hiervon ist.

6. Verwendung nach Anspruch 1, bei der die Stickstoff enthaltende heterocyclische Verbindung mindestens eine Verbindung ausgewählt aus Nikotinsäure und Nikotinsäureamid ist.

7. Verwendung nach einem der Ansprüche 1 bis 6, bei der die Menge an der eingearbeiteten Fluorid-Verbindung bei 50 bis 10000 µg pro Gramm der Zusammensetzung als gesamte Fluorkonzentration liegt und bei der das molare Verhältnis von Stickstoff enthaltender heterocyclischer Verbindung zur Fluorid-Verbindung bei 1/50 bis 10/1 liegt.

8. Verwendung nach einem der Ansprüche 1 bis 7, wobei die Zusammensetzung weiterhin aufweist (iii) mindestens ein Additiv, ausgewählt aus einem Poliermittel, Netzmittel, Verdickungsmittel, oberflächenaktiven Mittel, Öl, Neutralisiermittel, essentiellen Öl, Parfüm, Farbmittel, Konservierungsmittel, Antischimmelmittel, Antioxidationsmittel und Wasser.

## Revendications

1. Utilisation de:
   (i) au moins un dérivé de fluorure inorganique, et

(ii) au moins un dérivé hétérocyclique azoté sélectionné parmi pyridoxine, pyridoxal, pyridoxamine et leurs sels; 2.- ou 3-pyridilcarbinol; thiamine et ses sels; histidine et ses sels; et acide nicotinique et amide d'acide nicotinique;

pour la préparation d'un dentifrice ou d'une composition de bain de bouche permettant d'améliorer la capture du fluor dans les dents.

2. Utilisation selon la revendication 1, caractérisée en ce que le dérivé hétérocyclique azoté est au moins un dérivé sélectionné parmi pyridoxine, pyridoxal, pyridoxamine et leurs sels.

3. Utilisation selon la revendication 1, caractérisée en ce que le dérivé hétérocyclique azoté correspond au 2- ou 3-pyridylcarbinol.

4. Utilisation selon la revendication 1, caractérisée en ce que le dérivé hétérocyclique azoté correspond au moins à un dérivé sélectionné parmi la thiamine et ses sels.

5. Utilisation selon la revendication 1, caractérisée en ce que le dérivé hétérocyclique azoté correspond au moins à un dérivé sélectionné parmi l'histidine et ses sels.

6. Utilisation selon la revendication 1, caractérisée en ce que le dérivé hétérocyclique azoté correspond au moins à un dérivé sélectionné parmi l'acide nicotinique et l'amide d'acide nicotinique.

7. Utilisation selon l'une quelconque des revendications 1 à 6, caractérisée en ce que la quantité de dérivé de fluorure incorporée est de 50 à 10 000 $\mu$g par gramme de la composition en tant que concentration de fluor totale et le rapport molaire dérivé hétérocyclique azoté/dérivé de fluorure est de 1/50 à 10/1.

8. Utilisation selon l'une quelconque des revendications 1 à 7, caractérisée en ce que ladite composition comprend de plus:

(iii)au moins un additif sélectionné parmi: agent de polissage, agent mouillant, agent épaississant, tensioactif, huile, édulcorant, huile essentielle, colorant, parfum, conservateur, agent anti-moisissure, agent antioxydant et eau.